# EUROPÄISCHE PATENTSCHRIFT

(11) **EP 1 567 471 B1**
(45) Veröffentlichungstag und Bekanntmachung des Hinweises auf die Patenterteilung: **27.05.2009**
(21) Anmeldenummer: 03767556.8
(22) Anmeldetag: 17.11.2003
(51) Int. Cl.: C07C 57/07, C07C 57/075, C07C 45/86, C07C 45/82, C07C 67/48, C07C 69/533, C07C 47/22

(54) **VERFAHREN ZUR REKTIFIKATIVEN AUFTRENNUNG VON (METH)ACRYLMONOMERE ENTHALTENDEN FLÜSSIGKEITEN IN EINER REKTIFIKATIONSKOLONNE**
METHOD FOR THE RECTIFYING SEPARATION OF LIQUIDS CONTAINING (METH)ACRYLIC MONOMERS IN A RECTIFICATION COLUMN
PROCEDE POUR RECTIFIER PAR SEPARATION DES LIQUIDES CONTENANT DES MONOMERES (METH)ACRYLIQUES DANS UNE COLONNE DE RECTIFICATION

(30) Priorität: 29.11.2002 DE 10256147
(43) Veröffentlichungstag der Anmeldung: 31.08.2005
(73) Patentinhaber: BASF SE, 67056 Ludwigshafen (DE)
(72) Erfinder: SCHRÖDER, Jürgen, 67071 Ludwigshafen (DE)
(86) Internationale Anmeldenummer: PCT/EP2003/012815
(87) Internationale Veröffentlichungsnummer: WO 2004/050596

(56) Entgegenhaltungen:
- EP-A- 1 046 416
- DE-A- 19 501 325
- US-A- 4 260 821

## Beschreibung

Vorliegende Erfindung betrifft ein Verfahren der rektivikativen Auftrennung von (Meth)acrylmonomere enthaltenden Flüssigkeiten in einer Rektifikationskolonne, bei dem man während der Rektifikation aus der Rektifikationskolonne an wenigstens einer Entnahmestelle einen Stoffstrom entnimmt, den entnommenen Stoffstrom behandelt und nach der Behandlung wenigstens eine Teilmenge dieses Stoffstroms als flüssige Phase an wenigstens einer Rückführstelle in die Rektifikationskolonne rückführt.

Die Schreibweise (Meth)acrylmonomere steht in dieser Schrift verkürzend für "Acrylmonomere und/oder Methacrylmonomere".

Der Begriff Acrylmonomere steht in dieser Schrift verkürzend für "Acrolein, Acrylsäure und/oder Ester der Acrylsäure".

Der Begriff Methacrylmonomere steht in dieser Schrift verkürzend für "Methacrolein, Methacrylsäure und/oder Ester der Methacrylsäure.

Im besonderen sollen die in dieser Schrift angesprochenen (Meth)acrylmonomeren die nachfolgenden (Meth)acrylsäureester umfassen: Hydroxyethylacrylat, Hydroxyethylmethacrylat, Hydroxypropylacrylat, Hydroxypropylmethacrylat, Glycidylacrylat, Glycidylmethacrylat, Methylacrylat, Methylmethacrylat, n-Butylacrylat, iso-Butylacrylat, iso-Butylmethacrylat, n-Butylmethacrylat, tert.-Butylacrylat, tert.-Butylmethacrylat, Ethylacrylat, Ethylmethacrylat, 2-Ethylhexylacrylat, 2-Ethylhexylmethacrylat, N,N-Dimethylaminoethylacrylat und N,N-Dimethylaminoethylmethacrylat.

(Meth)acrylmonomere sind wichtige Ausgangsverbindungen zur Herstellung von Polymerisaten, die z.B. als Klebstoffe Verwendung finden.

(Meth)acrolein und (Meth)acrylsäure werden großtechnisch überwiegend durch katalytische Gasphasenoxidation geeigneter C₃-/C₄-Vorläuferverbindungen, insbesondere von Propen und Propan im Fall von Acrolein und Acrylsäure bzw. von iso-Buten und iso-Butan im Fall der Methacrylsäure und des Methacroleins, hergestellt. Neben Propen, Propan, iso-Buten und iso-Butan eignen sich als Ausgangsstoffe jedoch auch andere 3 bzw. 4 Kohlenstoffatome enthaltende Verbindungen, beispielsweise iso-Butanol, n-Propanol oder der Methylether (als C₄-Vorläufer) von iso-Butanol. (Meth)acrylsäure kann auch aus (Meth)acrolein erzeugt werden.

Dabei wird normalerweise ein Produktgasgemisch erhalten, aus dem die (Meth)acrylsäure bzw. das (Meth)acrolein abgetrennt werden muß.

Diese Abtrennung wird in der Regel so durchgeführt, dass die (Meth)acrylsäure bzw. das (Meth)acrolein durch Absorption in ein Lösungsmittel (z.B. Wasser oder ein organisches Lösungsmittel) oder durch fraktionierende Kondensation des Produktgasgemisches zunächst grundabgetrennt und das dabei anfallende Kondensat bzw. Absorbat nachfolgend rektifikativ (in der Regel in mehreren Stufen) unter Erhalt von mehr oder weniger reiner (Meth)acrylsäure bzw. (Meth)acrolein aufgetrennt wird (vgl. z.B. EP-A 717019, EP-A 1125912, EP-A 982289, EP-A 982287, DE-A 19606877, DE-A 1011527, DE-A 10224341, DE-A 10218419). Anstelle der fraktionierenden Kondensation kann auch zunächst eine Totalkondensation angewandt und das dabei anfallende Kondensat anschließend rektifikativ aufgetrennt werden.

Die vorstehend angesprochene fraktionierende Kondensation selbst soll in dieser Schrift nicht als unter den Begriff einer rektifikativen Auftrennung einer Flüssigkeit fallend betrachtet werden, unterscheidet sie sich von einer solchen Rektifikation doch dadurch, dass das aufzutrennende Gemisch der Trennkolonne nicht flüssig sondern gasförmig (d.h. vollständig in die Dampfform überführt) zugeführt wird. Im übrigen bewirkt bei einer Rektifikation der Stoffaustausch zwischen in der Rektifikationskolonne aufsteigendem Dampf und absteigender Rücklaufflüssigkeit die gewünschte Trennwirkung.

Ester der (Meth)acrylsäure sind z.B. durch direkte Umsetzung von (Meth)acrylsäure und/oder (Meth)acrolein mit den entsprechenden Alkoholen erhältlich. Allerdings fallen auch in diesem Fall zunächst in der Regel flüssige Produktgemische an, aus denen die (Meth)acrylsäureester z.B. rektifikativ abgetrennt werden müssen.

Die vorstehend angesprochenen, (Meth)acrylmonomere enthaltenden, Flüssigkeiten können die (Meth)acrylmonomere sowohl in mehr oder weniger reiner Form als auch in Lösung befindlich enthalten.

Das Lösungsmittel kann dabei sowohl wässrig als auch ein organisches Lösungsmittel sein. Die spezifische Art des Lösungsmittels ist für die vorliegende Erfindung im wesentlichen unbeachtlich. Der Gehalt an (Meth)acrylmonomeren kann ≥2, 5 Gew.-%, oder ≥10 Gew.-%, oder ≥20 Gew.-%, oder ≥40 Gew.-%, oder ≥60 Gew.-%, oder ≥80 Gew.-%, oder ≥90 Gew.-%, oder ≥95 Gew.-%, oder ≥99 Gew.-% betragen.

Die rektifikative Auftrennung der beschriebenen, (Meth)acrylmonomere enthaltenden, Flüssigkeiten kann je nach ihrer Zusammensetzung sowohl so erfolgen, dass sich die (Meth)acrylmonomere am Kopf der Rektifikationskolonne anreichern, als auch so, dass sich die (Meth)acrylmonomere im Sumpf der Rektifikationskolonne anreichern. Selbstredend können auch im oberen, unteren oder mittleren Teil der Rektifikationskolonne die (Meth)acrylmonomere angereichert enthaltenden Fraktionen entnommen werden.

In allen Fällen (insbesondere den vorgenannten) können zur in Rede stehenden rektifikativen Auftrennung Rektifikationskolonnen verwendet werden, die wenigstens einen Siebboden ohne Ablaufsegment enthalten (vgl. z.B. DE-A 19924532). Selbstverständlich können diese Rektifikationskolonnen aber auch ausschließlich Siebböden ohne Ablaufsegment als alleinige trennwirksame Einbauten der Rektifikationskolonne enthalten (vgl. z.B. DE-A 10156988 und EP-A 1029573). Es können aber auch andere trennwirksame Einbauten wie z.B. Glockenböden und/oder Packungen mitverwendet werden.

Solche Glockenböden und/oder Packungen können auch die alleinigen trennwirksamen Einbauten in der Rektifikationskolonne sein.

Die Rektifikation kann dabei sowohl unter Normaldruck als auch unter reduziertem Druck durchgeführt werden. Typische Sumpftemperaturen liegen im Bereich von 100 bis 250°C und typische Kopfdrucke betragen 80 bis 500 mbar.

(Meth)acrylmonomere häufig begleitende Lösungsmittel sind vielfach Diphenyl enthaltend, z.B. Mischungen aus Diphenylether, Diphenyl und o-Dimethylphthalat. Ein z.B. für die Absorption von (Meth)acrylmonomeren häufig verwendetes Lösungsmittel enthält ca. 57,4 Gew.-% Diphenylether, 20,7 Gew.-% Diphenyl und 20 Gew.-% o-Dimethylphthalat. Andere (Meth)acrylsäure und (Meth)acrylsäureester häufig begleitende Lösungsmittel sind Methylacrylat und Ethylacrylat.

In der Regel wird bei den in dieser Schrift zur Rede stehenden Rektifikationen die Rektifikationskolonne mittels sogenannter Polymerisationsinhibitoren polymerisationsinhibiert. Diese werden üblicherweise am Kopf der Kolonne aufgegeben, können zusätzlich aber auch dem Sumpf zugefügt werden und auch bereits in der aufzutrennenden, die (Meth)acrylmonomere enthaltenden, Flüssigkeit zugesetzt sein. Typische Vertreter solcher Polymerisationsinhibitoren sind Phenothiazin, 4-Methoxyphenol und 4-Hydroxy-2,2,6,6-tetramethylpiperidin-N-oxyl. Bezogen auf den Gehalt der (Meth)acrylmonomeren werden häufig bis zu wenigen hundert Gew.-ppm an Polymerisationsinhibitoren eingesetzt.

Ziel der Polymerisationsinhibierung ist es, eine unerwünschte Polymerisatbildung aufgrund unerwünschter Polymerisation der (Meth)acrylmonomere weitgehend zu unterdrücken, da solche Polymerisatbildung den Druckverlust über die Rektifikationskolonne in unerwünschter Weise erhöht und in extremen Fällen eine Unterbrechung der Rektifikation mit Waschung der Rektifikationskolonne erforderlich macht.

Es ist auch bekannt (vgl. z.B. DE-A 19501325 und Chem. Eng. Technol. 21 (1998) 10, S. 829 bis 836) zum Zweck einer zusätzlichen Polymerisationsinhibierung die Rektifikationskolonne während der Rektifikation mit einem molekularen Sauerstoff enthaltenen Gas zu durchströmen (vgl. auch DE-A 10238145).

Nachteilig an den beschriebenen Verfahren des Standes der Technik ist jedoch, dass die insgesamt erzielte polymerisationsinhibierende Wirkung nicht im vollen Umfang zu befriedigen vermag.

Als Verfahrensverbesserung wird in der DE-A 19501325 vorgeschlagen, die rektifikative Abtrennung von (Meth)acrylsäure aus einer (Meth)acrylsäure enthaltenden Flüssigkeit z.B. so durchzuführen, dass an wenigstens einer Stelle der Rektifikationskolonne in derselben absteigende Rücklaufflüssigkeit aus der Rektifikationskolonne herausgeführt, darin enthaltene oligo- und/oder polymerisierte (Meth)acrylsäure abgetrennt und anschließend die entnommene Rücklaufflüssigkeit in die Rektifikationskolonne zurückgeführt wird, doch vermag auch diese Verfahrensweise unter dem Aspekt der Polymerisationsinhibierung nicht völlig zu befriedigen.

Die Aufgabe der vorliegenden Erfindung bestand daher darin, die Verfahrensweise gemäß Stand der Technik zusätzlich zu verbessern.

Demgemäß wurde ein Verfahren der rektifikativen Auftrennung von (Meth)acrylmonomere enthaltenden Flüssigkeiten in einer Rektifikationskolonne, bei dem man während der Rektifikation aus der Rektifikationskolonne an wenigstens einer Entnahmestelle einen Stoffstrom entnimmt, den entnommenen Stoffstrom behandelt und nach der Behandlung wenigstens eine Teilmenge dieses Stoffstroms als flüssige Phase an wenigstens einer Rückführstelle in die Rektifikationskolonne rückführt, gefunden, das dadurch gekennzeichnet ist, dass der Gehalt G_{R} der in die Rektifikationskolonne rückgeführten flüssigen Phase an molekularem Sauerstoff, ausgedrückt in Prozenten des Gewichtes dieser Phase, wenigstens doppelt so hoch ist, wie der an der Rückführstelle in der Rücklaufflüssigkeit der Rektifikationskolonne vorliegende und in Prozenten des Gewichtes der Rücklaufflüssigkeit ausgedrückte Gehalt G_{F} an molekularem Sauerstoff.

Als Rektifikationskolonne kommen für das erfindungsgemäße Verfahren alle gängigen Typen in Betracht. D.h., die Rektifikationskolonne kann z.B. eine Boden-, Füllkörper-, oder Packungskolonne sein. Selbstredend können auch vorgenannte Einbauten in gemischter Form angewendet werden.

Als Füllkörperschüttungen können z.B. Ringe, Wendeln, Sattelkörper, Raschig-, Intos- oder Pall-Ringe, Barrel- oder Intalox-Sättel, Top-Pak oder Geflechte verwendet werden.

Als trennwirksame Böden kommen z.B. Glockenböden, Siebböden, Ventilböden, Tunnelböden, Thormannböden und/oder Dual-Flow-Böden in Betracht. Erfindungsgemäß bevorzugt werden Rektifikationskolonnen verwendet, die ausschließlich Dual-Flow-Böden als trennwirksame Einbauten enthalten (vgl. z.B. DE-A 10156988, EP-A 1029573 und DE-A 10230219).

Die erfindungsgemäß aufzutrennenden, (Meth)acrylmonomere enthaltenden Flüssigkeiten können die (Meth)acrylmonomere sowohl in mehr oder weniger reiner Form als auch in Lösung befindlich enthalten.

Das Lösungsmittel kann dabei sowohl wässrig als auch ein organisches Lösungsmittel sein. Die spezifische Art des Lösungsmittels ist erfindungsgemäß im wesentlichen unbeachtlich. Der Gehalt an (Meth)acrylmonomeren kann ≥2 Gew.-%, oder ≥5 Gew.-%, oder ≥10 Gew.-%, oder ≥20 Gew.-%, oder ≥40 Gew.-%, oder ≥60 Gew.-%, oder ≥80 Gew.-%, oder ≥90 Gew.-%, oder ≥95 Gew.-%, oder ≥99 Gew.-% betragen.

Die erfindungsgemäße rektifikative Auftrennung der beschriebenen (Meth)acrylmonomere enthaltenden Flüssigkeiten kann je nach ihrer Zusammensetzung sowohl so erfolgen, dass sich die (Meth)acrylmonomere am Kopf der Rektifikationskolonne anreichern, als auch so, dass sich die (Meth)acrylmonomere im Sumpf der Rektifikationskolonne anreichern. Selbstredend können auch im oberen, unteren oder mittleren Teil der Rektifikationskolonne die die (Meth)acrylmonomere angereichert enthaltenden Fraktionen entnommen werden.

In zweckmäßigerweise wird man das erfindungsgemäße Verfahren in einer aus Abtriebsteil und Verstärkerteil bestehenden Rektifikationskolonne durchführen.

D.h., die Einspeisung der rektifikativ aufzutrennenden Methacrylmonomere enthaltenden Flüssigkeit erfolgt vorzugsweise nicht im Sumpf, sondern an einer längs der Kolonne gelegenen Eintrittsstelle. Der unterhalb dieser Eintrittsstelle gelegene Teil der Rektifikationskolonne wird als Abtriebsteil und der oberhalb dieser Eintrittsstelle gelegene Teil der Rektifikationskolonne wird als Verstärkerteil bezeichnet. Mit Vorteil wird man in diesem Fall beim erfindungsgemäßen Verfahren als den erfindungsgemäß an wenigstens einer Entnahmestelle zu entnehmenden Stoffstrom in der Rektifikationskolonne absteigende Rücklaufflüssigkeit wählen und selbige oberhalb der Eintrittsstelle der erfindungsgemäß aufzutrennenden (Meth)acrylmonomere enthaltenden Flüssigkeit in die Rektifikationskolonne aus selbiger herausführen. Die Rückführung des erfindungsgemäß behandelten Stoffstroms als flüssige Phase in die Rektifikationskolonne erfolgt dann zweckmäßigerweise unmittelbar unterhalb der Entnahmestelle (an einer oder mehreren Rückführstellen). Insbesondere bei gasförmiger Entnahme des erfindungsgemäß zu behandelnden Stoffstroms, kann die Rückführung der flüssigen Phase aber auch auf der Höhe der Entnahmestelle erfolgen. Selbstverständlich kann das erfindungsgemäße Verfahren längs der Rektifikationskolonne mehrfach angewendet werden. Auch kann sich zwischen der Entnahmestelle und der Rückführstelle ein größerer Abstand befinden. Bei der vorstehend skizzierten erfindungsgemäßen Verfahrensweise wird man die abgetrennten (Meth)acrylmonomeren mit Vorzug oberhalb der Stelle, bei der die die aufzutrennende (Meth)acrylmonomere enthaltende Flüssigkeit in die Rektifikationskolonne eintritt, aus der Rektifikationskolonne entnehmen. Die wenigstens eine Entnahmestelle für den erfindungsgemäß zu entnehmenden Stoffstrom kann sich unterhalb und/oder oberhalb dieser Entnahmestelle befinden.

Für das erfindungsgemäße Verfahren erforderliche Wärmezufuhr erfolgt beim erfindungsgemäßen Verfahren in zweckmäßiger Weise z.B. über innen- und/oder außenliegende Wärmetauscher herkömmlicher Bauart und/oder Doppelwandheizung. Bevorzugt werden außenliegende Umlaufverdampfer mit Natur- oder Zwangsumlauf eingesetzt. Erfindungsgemäß besonders vorteilhaft sind außenliegende Umlaufverdampfer mit Zwangsumlauf. Der Einsatz mehrerer in Reihe oder parallel geschalteter Verdampfer ist erfindungsgemäß möglich.

Der beim erfindungsgemäßen Verfahren der Rektifikationskolonne an wenigstens einer Entnahmestelle zu entnehmende Stoffstrom kann prinzipiell sowohl ausschließlich Gasphase als auch ausschließlich Flüssigphase oder auch ein zweiphasiges Gemisch aus Gas- und Flüssigphase sein.

Die erfindungsgemäß durchzuführende Behandlung des entnommenen Stoffstroms kann verschiedenste Behandlungsschritte umfassen. Beispielsweise kann die in der DE-A 19501325 beschriebene Abtrennung von oligo- und/oder polymerisierten (Meth)acrylmonomeren einen solchen Behandlungsschritt bilden.

Besteht der entnommene Stoffstrom ausschließlich aus Gasphase, wird seine Behandlung im Rahmen des erfindungsgemäßen Verfahrens eine wenigstens teilweise (bevorzugt eine vollständige) Kondensation umfassen, muß erfindungsgemäß doch zwingend wenigstens eine Teilmenge des entnommenen Stoffstroms als flüssige Phase in die Rektifikationskolonne rückgeführt werden.

In der Regel wird die erfindungsgemäß durchzuführende Behandlung des entnommenen Stoffstroms auch die Behandlung mit einer Quelle von molekularem Sauerstoff umfassen, um zu gewährleisten, dass das Verhältnis G_{R}/G_{F} ≥2 erfindungsgemäß erfüllt wird.

Normalerweise wird man dabei als Quelle von molekularem Sauerstoff ein molekularen Sauerstoff enthaltendes Gas verwenden. Diese Quelle könnte reiner molekularer Sauerstoff oder ein Gemisch aus molekularem Sauerstoff und einem vorzugsweise inerten Gas (z.B. N₂) sein. Besonders bevorzugt wird Luft verwendet.

Wird als erfindungsgemäß zu entnehmender Stoffstrom Rücklaufflüssigkeit entnommen, so wird diese in zweckmäßiger Weise einem Verweilzeitgefäß zugeführt. Aus diesem Verweilzeitgefäß wird dann üblicherweise eine Teilmenge der enthaltenen flüssige Phase entnommen und wie erfindungsgemäß gefordert an wenigstens einer Rückführstelle in die Rektifikationkolonne rückgeführt, die bevorzugt unterhalb der Entnahmestelle liegt. Eine andere Teilmenge der im Verweilzeitgefäß enthaltenen flüssigen Phase wird ebenfalls entnommen, gegebenenfalls (bevorzugt nicht) gekühlt und anschließend über eine Sprühvorrichtung in das Verweilzeitgefäß rückversprüht. Gleichzeitig wird dem Verweilzeitgefäß z.B. Luft zugeführt, aus welcher die rückversprühten Tröpfchen der flüssigen Phase molekularen Sauerstoff aufnehmen und sich mit diesem anreichen. Die an molekularem Sauerstoff verarmte Luft kann z.B. über ein mit einem Rückflusskühler versehenes Abluftrohr weggeführt werden.

Die mittlere Verweilzeit der flüssigen Phase im Verweilzeitgefäß kann in typischer Weise 0,5 bis 10 h betragen. Erfindungsgemäß bevorzugt wird die Verweilzeit im Verweilzeitgefäß und der Luftstrom so bemessen, dass sich die flüssige Phase unter den gegebenen Randbedingungen mit molekularem Sauerstoff sättigt, bevor sie an wenigstens einer Rückführstelle in die Rektifikationskolonne rückgeführt wird.

Es können so Verhältnisse G_{R}/G_{F} von ≥3, oder ≥5, oder ≥10 oder ≥20, oder ≥50 erzielt werden.

Die vorstehend beschriebene Entnahme von Rücklaufflüssigkeit aus der Rektifikationskolonne kann auf verschiedene Arten erfolgen. Beispielsweise über einen Kaminboden, wie er in der DE-A 10159615 beschrieben ist, oder über einen Abzugsboden, wie ihn die DE-A 4231081 beschreibt.

Selbstverständlich kann der der Rektifikationskolonne erfindungsgemäß an wenigstens einer Entnahmestelle zu entnehmende Stoffstrom auch Brüden sein. In diesem Fall wird der Brüden vorteilhaft einem Sättigersystem zugeführt und in diesem mittels eines direkt oder indirekt kühlenden Kühlers wenigstens partiell kondensiert. In diesem Fall kann auf einen speziellen Abzugs- und/oder Kaminboden verzichtet werden.

Als Sättiger ist jeder Behälter geeignet, der für den entsprechenden Arbeitsdruck und die entsprechende Arbeitstemperatur ausgelegt ist. Bevorzugt werden Sättiger verwendet, die höher als breit sind. Am Boden des Sättigers wird eine erste Teilmenge an Kondensat entnommen (das durch indirekte und/oder direkte Kühlung erzeugt wurde) und im oberen Bereich des Sättigers, gegebenenfalls nach vorheriger Abkühlung durch indirekten Wärmetausch, verdüst. Erfindungsgemäß zweckmäßig wird dem Kondensat vorab seiner Versprühung Polymerisationsinhibitor zugesetzt.

Gleichzeitig wird z.B. mit Vorteil, wie z.B. im Fall des Verweilzeitgefäßes, Luft zugeführt. Die indirekte Kühlung zu Kondensationszwecken kann im Sättiger beispielsweise durch einen Rohrbündelwärmetauscher bewirkt werden.

Wie erfindungsgemäß gefordert, wird eine zweite Teilmenge an mit molekularem Sauerstoff angereichertem Kondensat an wenigstens einer Rückführstelle in die Rektifikationskolonne rückgeführt.

Die Temperatur im Verweilzeitgefäß und im Sättiger unterliegt keinen Beschränkungen. Vorzugsweise wird im Sättiger eine Temperatur gewählt, die eine indirekte Kühlung im Sättiger neben der direkten Kühlung entbehrlich macht. Der Druck im Verweilzeitgefäß und im Sättiger unterliegt ebenfalls keinen Beschränkungen. Bevorzugt ist dieser Druck nicht niedriger als an der Entnahmestelle. Ganz besonders bevorzugt werden Verweilzeitgefäß und Sättiger bei Normaldruck betrieben.

Das sauerstoffhaltige Gas wird erfindungsgemäß vorteilhaft in einer Menge von 0,1 bis 1000 Nm³ (bevorzugt 1 bis 100 m³) pro Stunde und m³ Volumen des Sättigers in den Sättiger dosiert. Vorzugsweise beträgt sein Gehalt an molekularem Sauerstoff 1 bis 50 mol-%, besonders bevorzugt 5 bis 25 mol-%. Bei aus der Rektifikationskolonne herausgeführten Brüden mit einem Flammpunkt nach DIN 51755 von ≤50°C weist das molekularen Sauerstoff enthaltende Gas bevorzugt einen molekularen Sauerstoffgehalt von 4 bis 10 mol-% auf.

Normalerweise ist der Partialdruck an molekularem Sauerstoff im Sättiger (P_{OS}) größer als der Partialdruck an molekularem Sauerstoff an der Rückführstelle (P_{OR}). Günstig ist es wenn gilt P_{OS}/P_{OR} ≥2, oder ≥10. Das Vorgenannte gilt so im wesentlichen auch im Fall des Verweilzeitgefäßes.

Ist die Rektifikationskolonne eine Bodenkolonne, erfolgt die erfindungsgemäße Rückführung der erfindungsgemäß behandelten flüssigen Phase bevorzugt auf den Boden unmittelbar unter der Entnahmestelle.

Als Quelle für den molekularen Sauerstoff kann anstelle eines molekularen Sauerstoff enthaltenden Gases aber auch z.B. ein molekularen Sauerstoff freisetzendes System im Sättiger oder im Verweilzeitgefäß angewandt werden, vorausgesetzt, dieses setzt keinen radikalischen Sauerstoff frei.

Eine elegante Ausführung des erfindungsgemäßen Verfahrens besteht darin, die Rektifkationskolonne mit einem molekularen Sauerstoff enthaltenden Gas, z.B. Luft, von unten nach oben und/oder von oben nach unten zu durchströmen. Auf diese Art und Weise enthält auch entnommener Brüden bereits molekularen Sauerstoff. Aufgrund der im Sättiger einhergehenden wenigstens partiellen Kondensation, steigt der Partialdruck an molekularem Sauerstoff im Sättiger sprunghaft an. Dieser erhöhte Sauerstoffpartialdruck führt zu einer Sauerstoffanreicherung im Kondensat und damit in der rückzuführenden flüssigen Phase, wie sie erfindungsgemäß erwünscht ist. Nicht kondensierbare Anteile (z.B. N₂) können wiederum durch ein mit Rückflußkühler versehenes Abgasrohr aus dem Sättiger geführt werden. ,

In überraschender Weise ist die polymerisationsinhibierende Wirkung des in der erfindungsgemäß in die Rektifikationskolonne rückgeführten flüssigen Phase enthaltenen molekularen Sauerstoff noch in einem größeren Kolonnenbereich unterhalb der Rückführstelle spürbar, obwohl die Konzentration des in dieser flüssigen Phase gelösten Sauerstoffs signifikant über dem thermodynamischen Gleichgewichtspartialdruck in der Rektifikationskolonne liegt und die Stoffaustauschprozesse in einer Rektifikationskolonne sehr schnell sind.

Natürlich wird man auch beim erfindungsgemäßen Verfahren die Rektifikationskolonne bevorzugt polymerisationsinhibiert betreiben. Diese Polymerisationsinhibitoren, die in der Regel am Kopf der Kolonne aufgegeben werden, können zusätzlich aber auch dem Sumpf zugeführt werden und auch bereits in der erfindungsgemäß aufzutrennenden, die (Meth)acrylmonomere enthaltenden, Flüssigkeit zugesetzt sein.

Als Polymerisationsinhibitor können hier z.B. Alkylphenole, beispielsweise o-, m- oder p-Kresol (Methylphenol), 2-tert.-Butyl-4-methylphenol, 6-tert.-Butyl-2,4-dimethylPhenol, 2,6-Di-tert.-Butyl-4-methylphenol, 2-tert.-Butylphenol, 4-tert.-Butylphenol, 2,4-di-tert.-Butylphenol, 2-Methyl-4-tert.-Butylphenol, 4-tert.-Butyl-2,6-dimethylphenol, oder 2,2'-Methylen-bis-(6-tert.-butyl-4-methylphenol), Hydroxyphenole, beispielsweise Hydrochinon, 2-Methylhydrochinon, 2,5-Di-tert.-Butylhydrochinon, Brenzcatechin (1,2-Dihydroxybenzol) oder Benzochinon, Aminophenole, wie z.B. para-Aminophenol, Nitrosophenole, wie z.B. para-Nitrosophenol, Alkoxyphenole, beispielsweise 2-Methoxyphenol (Guajacol, Brenzcatechinmonomethylether), 2-Ethoxyphenol, 2-Isopropoxyphenol, 4-Methoxyphenol (Hydrochinonmonomethylether), Mono- oder Di-tert.-Butyl-4-methoxyphenol, Tocopherole sowie 2,3-Dihydro-2,2-dimethyl-7-hydroxybenzofuran (2,2-Dimethyl-7-hydroxycumaran), N-Oxyle, wie 4-Hydroxy-2,2,6,6-tetramethyl-piperidin-N-oxyl, 4-Oxo-2,2,6,6-tetramethyl-piperidin-N-oxyl, 4-Acetoxy-2,2,6,6-tetramethyl-piperidin-N-oxyl, 2,2,6,6-Tetramethyl-piperidin-N-oxyl, 4,4',4"-Tris(2,2,6,6-tetramethylpiperidin-N-oxyl)-phosphit oder 3-Oxo-2,2,5,5-tetramethylpyrrolidin-N-oxyl, aromatische Amine oder Phenylendiamine, wie z.B. N,N-Diphenylamin, N-Nitrosodiphenylamin, N,N'-Dialkyl-paraphenylendiamin, wobei die Alkylreste gleich oder verschieden sein können und jeweils unabhängig voneinander 1 bis 4 Kohlenstoffatomen enthalten und geradkettig oder verzweigt sein können, Hydroxylamine, wie z.B. N,N-Diethylhydroxylamin, phosphorhaltige Verbindungen, wie z.B. Triphenylphosphin, Triphenylphosphit, Hypophosphorige Säure oder Triethylphosphit, schwefelhaltige Verbindungen, wie z.B. Diphenylsulfid oder Phenothiazin, gegebenenfalls in Kombination mit Metallsalzen, wie beispielsweise die Chloride, Dithiocarbamate, Sulfate, Salicylate oder Acetate von Kupfer, Mangan, Cer, Nickel oder Chrom verwendet werden. Selbstverständlich können auch Gemische von Stabilisatoren eingesetzt werden.

Erfindungsgemäß bevorzugt wird, insbesondere im Fall von Acrylsäure als (Meth)acrylmonomerem, Phenothiazin, Hydrochinonmonomethylether und/oder N,N-Di-sec.-butyl-p-phenylendiamin verwendet. Die Konzentration des verwendeten Polymerisationsinhibitorsystems beträgt in allen Flüssigphasen vorteilhaft 100 bis 5000, vorzugsweise 200 bis 1000 gew.ppm, bezogen auf das Gewicht der Flüssigphase.

Die Art der Stabilisatorzugabe unterliegt ebenfalls keiner Beschränkung. Vorzugsweise wird der Stabilisator (Polymerisationsinhibitor) als Lösung oder in Substanz in den oberen Bereich der Rektifikationskolonne dosiert. Die Abkühlung der Leichtsiederfraktion zum Zwecke deren Kondensation kann im Kopfbereich beispielsweise durch indirekte Wärmetauscher, die dem Fachmann an sich bekannt sind und keiner Beschränkung unterliegen, oder direkt, beispielsweise durch einen Quench, erfolgen. Vorzugsweise wird die direkte Kühlung angewendet. Dazu wird bereits kondensierte Leichtsiederfraktion mittels eines geeigneten Wärmetauschers gekühlt und das gekühlte Kondensat oberhalb der Abnahmestelle im Brüden versprüht. Die Kondensation der Leichtsiederfraktion kann auch mehrstufig ausgeführt sein. Bei Verwendung einer indirekten Kühlung wird der Stabilisator bevorzugt von oben auf die Kühleinheit versprüht (z.B. in die Brüden durchströmten Rohre eines Rohrbündelwärmetauschers). Bei Verwendung einer direkten Kühlung wird der Polymerisationsinhibitor bevorzugt in den Quenchkreis dosiert. In beiden Fällen wird die Rektifikationskolonne über den Rücklauf stabilisiert.

Zur weiteren und/oder in extremen Fällen alleinigen Unterstützung der erfindungsgemäßen Verfahrensweise wird zweckmäßig ein (molekularen) sauerstoffhaltiges Gas, bevorzugt Luft oder ein Gemisch aus Luft und Stickstoff (Magerluft) durch die Rektifikationskolonne geführt. Dieses sauerstoffhaltige Gas wird vorzugsweise in den Sumpfbereich der Rektifikationseinheit und/oder in einen mit diesem verbundenen Umlaufverdampfer eindosiert.

Selbstverständlich kann das erfindungsgemäße Verfahren auch mit allen anderen Verfahren des Standes der Technik zur Unterdrückung von Polymerisatbildung gekoppelt angewendet werden. Das sind insbesondere jene der DE-A 19501325, der DE-A 10238145, der DE-A 10217121, der DE-A 10139767 und der DE-A 10223618.

In besonders effizienter Weise lässt sich das erfindungsgemäße Verfahren dann verwirklichen, wenn das rektifikative Verfahren die Abtrennung von (Meth)acrylsäure aus einem (Meth)acrylsäure und eine höher als (Meth)acrylsäure siedende inerte hydrophobe organische Flüssigkeit als Hauptbestandteile sowie niedere Aldehyde als Nebenbestandteile enthaltenden Gemisch betrifft, wie es z. B. im Rahmen der Abtrennung von (Meth)acrylsäure aus dem Reaktionsgasgemisch der katalytischen Gasphasenoxidation gemäß den in der DE-A 44 36 243, der DE-PS 21 36 396, der EP-A 925 272 und der DE-A 43 08 087 beschriebenen Verfahrensweisen auftritt. D.h., wenn das (Meth)acrylsäure und eine höher als (Meth)acrylsäure siedende inerte hydrophobe organische Flüssigkeit als Hauptbestandteile sowie niedere Aldehyde als Nebenbestandteile enthaltende Einsatzgemisch für das erfindungsgemäße Verfahren z.B. aus den Reaktionsgasgemischen der katalytischen Gasphasenoxidation als Flüssigkeitsablauf einer Gegenstromabsorption mit anschließender Desorption durch Abstreifen gemäß der DE-PS 21 36 39, der EP-A 925 272 oder der DE-A 43 08 087 oder als Flüssigkeitsablauf einer Gegenstromabsorption mit überlagerter Rektifikation gemäß der DE-A 44 36 243 erhalten wurde. Unter hochsiedender inerter hydrophober organischer Flüssigkeit sind hier solche Flüssigkeiten zu verstehen, deren Siedepunkt bei Normaldruck (1 atm) oberhalb der Siedetemperatur der (Meth)acrylsäure liegt und in denen die Löslichkeit (Gew.-%, bezogen auf das Gewicht der Lösung) von (Meth)acrylsäureoligo- und/oder -polymerisaten bei 25°C und 1 atm geringer als in reiner (Meth)acrylsäure ist.

Insbesondere sind es solche hochsiedenden organischen Flüssigkeiten, die zu wenigstens 70 Gew:-% aus solchen Molekülen bestehen, die keine nach außen wirkende polare Gruppe enthalten und somit beispielsweise nicht in der Lage sind, Wasserstoffbrücken zu bilden. Im engeren Sinn umfasst der Begriff hier die hochsiedenden organischen Absorptionsflüssigkeiten, die in der DE-PS 21 36 396, der DE-A 43 08 087 sowie der DE-A 44 36 243 empfohlen werden.

Dies sind im wesentlichen Flüssigkeiten, deren Siedepunkt bei Normaldruck oberhalb von 160°C liegt. Beispielhaft genannt seien Mittelölfraktionen aus.der Paraffindestillation, Diphenylether, Diphenyl oder Mischungen der vorgenannten Flüssigkeiten wie z.B. ein Gemisch aus 70 bis 75 Gew.-% Diphenylether und 25 bis 30 Gew.-% Diphenyl (Diphyl genannt). Eine günstige hochsiedende hydrophobe organische Absorptionsflüssigkeit ist auch ein Gemisch, bestehend aus einer Mischung aus 70 bis 75 Gew.-% Diphenylether und 25 bis 30 Gew.-% Diphenyl, sowie, bezogen auf diese Mischung, 0,1 bis 42,5 Gew.-% o-Dimethylphthalat.

Im Fall der Methacrylsäure kann dabei die gasphasenkatalytisch oxidative Herstellung z.B. ausgehend von Methacrolein erfolgt sein, das seinerseits durch gasphasenkatalytische Oxidation von tert.-Butanol, iso-Butan oder iso-Buten oder durch Umsetzung von Formaldehyd mit Propionaldehyd gemäß der EP-B 92 097 oder der EP-B 58 927 erhalten worden sein kann. Häufig erfolgt die gasphasenkatalytische Oxidation des tert.-Butanols, iso-Butans oder iso-Butens unter Verwendung einer katalytisch aktiven Masse der allgemeinen Formel I

Mo₁₂BiₐFe_{b}X_{c}¹X_{d}²Xₑ³X_{f}⁴Oₙ (I),

in der die Variablen folgende Bedeutung haben:
- X¹: Nickel und/oder Kobalt,
- X²: Thallium, ein Alkalimetall und/oder ein Erdalkalimetall,
- X³: Phosphor, Arsen, Bor, Antimon, Zinn, Cer, Blei, Niob und/oder Wolfram,
- X⁴: Silicium, Aluminium, Titan und/oder Zirkonium,
- a: 0,5 bis 5,
- b: 0,01 bis 3,
- c: 3 bis 30,
- d: 0,02 bis 2,
- e: 0 bis 5,
- f: 0 bis 10 und
- n: eine ganze Zahl, die durch die Wertigkeit und Häufigkeit der von Sauerstoff verschiedenen Elemente in I bestimmt wird,
bei Temperaturen von 300 bis 400°C und, vom speziellen Temperaturverlauf abgesehen, ansonsten gemäß den Bedingungen der DE-A 40 23 239. Das dabei anfallende Methacrolein wird in der Regel ohne Zwischenreinigung zur Weiteroxidation verwendet. Dabei kann die gasphasenkatalytische Oxidation des Methacroleins, vom speziellen Temperaturverlauf abgesehen, gemäß den Ausführungen der DE-A 41 32 263 bei Temperaturen von 200 bis 350°C bzw. gemäß der DE-A 41 32 684 5o bei Temperaturen von 250 bis 400°C erfolgen.

Insbesondere können dabei die in der DE-A 40 22 212 aufgeführten Multimetalloxidkatalysatoren angewendet werden.

Im Fall von Acrylsäure kann die gasphasenkatalytisch oxidative Herstellung z.B. einstufig ausgehend von Acrolein oder zweistufig ausgehend von Propylen über Acrolein erfolgt sein. Als Multimetalloxidkatalysatoren kommen dabei für die katalytische Gasphasenoxidation des Propylens insbesondere solche der allgemeinen Formel II

Mo₁₂BiₐFe_{b}X_{c}¹X_{d}²Xₑ³X_{f}⁴Oₙ (II),

in der die Variablen nachfolgende Bedeutung aufweisen:
- X¹: Nickel und/oder Kobalt,
- X²: Thallium, ein Alkalimetall und/oder ein Erdalkalimetall,
- X³: Phosphor, Arsen, Bor, Antimon, Zinn, Cer, Blei, Niob und/oder Wolfram,
- X⁴: Silicium, Aluminium, Titan und/oder Zirkonium,
- a: 0,5 bis 5,
- b: 0,01 bis 3,
- c: 3 bis 10,
- d: 0,02 bis 2,
- e: 0 bis 5,
- f: 0 bis 10 und
- n: eine ganze Zahl, die durch die Wertigkeit und Häufigkeit der von Sauerstoff verschiedenen Elemente bestimmt wird,
und für die katalytische Gasphasenoxidation des Acroleins insbesondere solche der allgemeinen Formel III

Mo₁₂VₐW_{b}Cu_{c}Ni_{d}Xₑ¹X_{f}²X_{g}³Xₕ⁴Xᵢ⁵Oₙ (III),

in der die Variablen nachfolgende Bedeutung aufweisen:
- X¹: ein oder mehrere Alkalimetalle,
- X²: ein oder mehrere Erdalkalimetalle,
- X³: Chrom, Mangan, Cer und/oder Niob,
- X⁴: Antimon und/oder Wismut,
- X⁵: Silicium, Aluminium, Titan und/oder Zirkonium,
- a: 1 bis 6,
- b: 0,2 bis 4,
- c: 0,5 bis 6,
- d: 0,2 bis 6,
- e: 0 bis 2,
- f: 0 bis 3,
- g: 0 bis 5,
- h: 0 bis 40,
- i: 0 bis 40 und
- n: eine ganze Zahl, die durch die Wertigkeit und Häufigkeit der von Sauerstoff verschiedenen Elemente bestimmt wird,
in Betracht. Die Reaktionsgase der ersten Oxidationsstufe werden üblicherweise ohne Zwischenreinigung der zweiten Oxidationsstufe zugeführt. Die üblicherweise angewendeten Reaktionsbedingungen können z.B. der DE-A 44 31957 sowie der DE-A 44 31949 entnommen werden.

In der Regel enthält ein solches, wie vorstehend beschriebenes, im wesentlichen aus (Meth)acrylsäure und einer höher als (Meth)acrylsäure siedenden inerten hydrophoben organischen Flüssigkeit als Hauptbestandteilen sowie niederen Aldehyden als Nebenbestandteilen bestehendes Gemisch, 5 bis 25, meist 5 bis 15 Gew.-% (Meth)acrylsäure.

Die rektifikative Abtrennung von (Meth)acrylsäure über Kopf- oder Seitenabzug der Rektifikationskolonne erfolgt vorzugsweise bei reduziertem Druck (zweckmäßigerweise bei einem Kopfdruck ≤500 mbar, üblicherweise 10 bis 500 mbar, häufig 10 bis 200 mbar und vorzugsweise 10 bis 100 mbar; in entsprechender Weise betragen die Sumpftemperaturen in der Regel 100 bis 230°C).

### Beispiel und Vergleichsbeispiel

### a) Beispiel

Einer Glockenbodenkolonne, die 35 äquidistante Glockenböden und eine Länge von 3245 mm sowie einen Innendurchmesser von 80 mm aufwies, wurde eine wie im Ausführungsbeispiel der DE-A 195 01 325 erzeugte, Acrylsäure enthaltende, Flüssigkeit zugeführt, die folgende Mengenanteile enthielt:
17 Gew.-% Acrylsäure,
0,02 Gew.-% Wasser,
0,002 Gew.-% Acrolein,
0,002 Gew.-% Allylacrylat,
0,01 Gew.-% Furfural,
0,03 Gew.-% Essigsäure,
0,2 Gew.-% Benzaldehyd,
0,003 Gew.-% Propionsäure,
0,03 Gew.-% Maleinsäureanhydrid,
58 Gew.-% Diphyl,
17 Gew.-% Dimethylphthalat,
3 Gew.-% Acrolylpropionsäure und
200 Gew.-ppm Phenothiazin.

Die Glockenbodenkolonne war mit einem Doppelmantel ausgerüstet.

Die Temperatur des Doppelmantels wurde mittels eines Wärmeträgeröls auf 150°C (Boden 1 bis 11), 119°C (Boden 12 bis 25) und 100°C (Boden 26 bis 35) eingestellt.

Der Zulauf der aufzutrennenden Flüssigkeit erfolgte mit einer Temperatur von 20°C und einer Menge von 3250 g/h auf den 11. Boden der Rektifikationskolonne. Die Sumpftemperatur betrug 160°C und die Kopftemperatur betrug 76°C. Die Rektifikationskolonne wurde bei einem Kopfdruck von 80 mbar betrieben.

Die Sumpfflüssigkeit der Rektifikationskolonne wurde mit einem Zwangsumlaufverdampfer auf 160°C erwärmt, die Umlaufmenge betrug 500 l/h. Zusätzlich wurden 5000 Ncm³ Luft in den Verdampfer dosiert.

Die aufzutrennende Acrylsäure enthaltende Flüssigkeit wurde in der Rektifikationskolonne in eine 99,6 gew.-%ige Acrylsäure (Reinprodukt), ein Leichtsiedergemisch (leichter als Acrylsäure siedend) mit einem Acrylsäuregehalt von 96 Gew.-% und ein Schwersiedergemisch mit einem Acrlysäuregehalt von 0,5 Gew.-% aufgetrennt. Der Leichtsiederbrüden der Rektifikationskolonne wurde in einem Quenchkühler kondensiert. Der Quenchkühler war ein Leerrohr mit einer Länge von 700 mm und einem Innendurchmesser von 80 mm. Bereits kondensierter Brüden wurde mittels einer Pumpe in den oberen Teil des Leerrohres eingedüst. Die Förderleistung betrug 1000 l/h. Mittels eines Wärmetauschers im Pumpenkreis wurde die Temperatur im Quenchkühler auf 17°C eingestellt. Die Flüssigphase im Quenchkühler wurde mit 200 ml/h einer 0,3 gew.-igen Lösung von Phentothiazin in Reinprodukt inhibiert. Stündlich wurden 150 ml Leichtsiederkondensat ausgeschleust und 3600 ml Leichtsiederkondensat als Rücklauf verwendet. Aus dem Sumpf der Rektifikationskolonne wurden stündlich 2800 ml Sumpfflüssigkeit (Schwersiedergemisch) ausgeschleust.

Über einen Seitenabzug auf dem 25. Boden wurden stündlich 1400 g Acrylsäure (Reinprodukt) aus der Rektifikationskolonne herausgeführt. 600 g/h dieses Reinproduktes wurden einer Lagermöglichkeit zugeführt. Die anderen 800 g/h Reinprodukt wurden mit einer Temperatur von 60°C über ein Verweilzeitgefäß (2 I Innenvolumen) einem Sättiger zugeführt. Der Sättiger war ein zur Atmosphäre offenes Leerrohr (700 mm Länge, 80 mm Innendurchmesser). Mittels einer Pumpe wurde die Reinproduktacrylsäure aus dem Verweilzeitgefäß wiederkehrend in den oberen Teil des Leerrohres eingedüst. Die Förderleistung betrug 1000 l/h. Der Sättiger wurde gleichzeitig im Gleichstrom von 10000 Ncm³ Luft/h durchströmt. Der Druck im Sättiger betrug 1000 mbar.

Der Ablauf aus dem Sättiger wurde dem Verweilzeitgefäß zugeführt. Aus dem Sättiger, der einen Flüssigkeits-hold-up von 800 ml aufwies, wurden 800 g/h an Flüssigphase auf den 24. Glockenboden der Rektifikationskolonne rückgeführt. Sie enthielt 45 Gew.-ppm O₂. Die dem 24. Glockenboden zulaufende Rücklaufflüssigkeit enthielt < 1 Gew.-ppm O₂.
Die Rektifikationskolonne wurde 40 Tage ohne Beeinträchtigung betrieben.

### b) Vergleichsbeispiel

Es wurde wie im Beispiel verfahren. Die 800 g/h Reinprodukt wurden jedoch unter Umgehung des Sättigers nach Entnahme vom 25. Glockenboden auf den 24. Glockenboden geführt (über ein entsprechendes Rohr). Nach einer Laufzeit von 19 Tagen musste die Rektifikation abgebrochen werden. Im Bereich der Böden 22 bis 24 waren die Glockenböden infolge Polymerisatbildung verstopft.

## Patentansprüche

1. Verfahren zur rektifikativen Auftrennung von (Meth)acrylmonomere enthaltenden Flüssigkeiten in einer Rektifikationskolonne, bei dem man während der Rektifikation aus der Rektifikationskolonne an wenigstens einer Entnahmestelle einen Stoffstrom entnimmt, den entnommenen Stoffstrom behandelt und nach der Behandlung wenigstens eine Teilmenge dieses Stoffstroms als flüssige Phase an wenigstens einer Rückführstelle in die Rektifikationskolonne rückführt, **dadurch gekennzeichnet, dass** der Gehalt G_{R} der in die Rektifikationskolonne rückgeführten flüssigen Phase an molekularem Sauerstoff, ausgedrückt in Prozenten des Gewichtes dieser Phase, wenigstens doppelt so hoch ist, wie der an der Rückführstelle in der Rücklaufflüssigkeit der Rektifikationskolonne vorliegende und in Prozenten des Gewichtes der Rücklaufflüssigkeit ausgedrückte Gehalt G_{F} an molekularem Sauerstoff.

2. Verfahren nach Anspruch 1, **dadurch gekennzeichnet, dass** das Verhältnis G_{R}/G_{F} ≥5 beträgt.

3. Verfahren nach Anspruch 1, **dadurch gekennzeichnet, dass** das Verhältnis G_{R}/G_{F} ≥20 beträgt.

## Claims

1. A process for rectificatively separating liquids comprising (meth)acrylic monomers in a rectification column by withdrawing a stream from the rectification column at at least one withdrawal point during rectification, treading the stream withdrawn and, after the treatment, recycling at least a portion of this stream as a liquid phase into the rectification column at at least one recycle point, wherein the content C_{R} in the liquid phase recycled into the rectification column of molecular oxygen, expressed in percent of the weight of this phase, is at least twice as high as the content C_{F} of molecular oxygen present in the reflux liquid of the rectification column at the recycle point and expressed in percent of the weight of the reflux liquid.

2. The process according to claim 1, wherein the C_{R}/C_{F} ratio is ≥ 5.

3. The process according to claim 1, wherein the C_{R}/C_{F} ratio is ≥ 20.

## Revendications

1. Procédé de séparation par rectification de liquides contenant des monomères (méth)acryliques dans une colonne de rectification, dans lequel un courant de substance est soutiré de la colonne de rectification en au moins un point de soutirage durant la rectification, le courant de substance soutiré est traité et, après le traitement, au moins une quantité partielle de ce courant de substance est recyclée dans la colonne de rectification en tant que phase liquide en au moins un point de recyclage, **caractérisé en ce que** la quantité G_{R} d'oxygène moléculaire dans la phase liquide recyclée dans la colonne de rectification, exprimée en pourcentage du poids de cette phase, correspond au moins au double de la quantité G_{F} d'oxygène moléculaire présente au point de recyclage dans le liquide de reflux de la colonne de rectification et exprimée en pourcentage du poids du liquide de reflux.

2. Procédé selon la revendication 1, **caractérisé en ce que** le rapport G_{R}/G_{F} est ≥5.

3. procédé selon la revendication 1, **caractérisé en ce que** le rapport G_{R}/G_{F} est ≥20.
